# EUROPÄISCHE PATENTSCHRIFT

(11) **EP 1 442 713 B1**
(45) Veröffentlichungstag und Bekanntmachung des Hinweises auf die Patenterteilung: **03.05.2006**
(21) Anmeldenummer: 03029327.8
(22) Anmeldetag: 19.12.2003
(51) Int. Cl.: A61B 17/16

(54) **Bohrwerkzeug für Knochen, insbesondere den proximalen Femur**
Drill for bones, in particular for the proximal femur
Foret pour os, notamment pour le fémur proximal

(30) Priorität: 23.01.2003 DE 20300988 U
(43) Veröffentlichungstag der Anmeldung: 04.08.2004
(73) Patentinhaber: Stryker Trauma GmbH, 24232 Schönkirchen (DE)
(72) Erfinder: Simon, Bernd, Dipl.-Ing., 24106 Kiel (DE); Völzow, Stefan, 24248 Mönckeberg (DE)
(74) Vertreter: Graalfs, Edo

(56) Entgegenhaltungen:
- EP-A- 0 861 635
- WO-A-00/45714
- US-A- 5 171 248

## Beschreibung

Die Erfindung bezieht sich auf ein Bohrwerkzeug für einen Knochen, insbesondere den proximalen Femur nach dem Oberhegriff des Patentanspruchs 1.

Es ist bekannt, zur Versorgung von Femurfrakturen, insbesondere des proximalen Femurs, Schenkelhalsstifte, insbesondere Schenkelhalsschrauben zu verwenden. Sie werden entweder in Verbindung mit einer sogenannten Pohl'schen Lasche eingesetzt oder einem Verriegelungsnagel. Im ersteren Fall enthält eine extramedullär am Knochen angebrachte Lasche eine Hülse, durch welche die Schenkelhalsschraube geführt ist. Im zweiten Fall weist der Verriegelungsnagel eine Schrägbohrung auf zur Aufnahme der Schenkelhalsschraube.

Um die Schenkelhalsschraube sicher im Knochen zu verankern, wird der Knochen üblicherweise auf den Kerngewindedurchmesser aufgebohrt, bevor die Schenkelhalsschraube eingedreht wird.

Bevor ein derartiger Bohrvorgang erfolgt, ist zunächst die Bohrachse und damit die Lage der Schenkelhalsschraube im Knochen unter Röntgenkontrolle zu bestimmen. Dies geschieht üblicherweise mit einem sogenannten Kirschner-Draht. Nach Ermittlung der richtigen Position verbleibt der Kirschner-Draht im Knochen und dient als Führung für das Bohrwerkzeug und anschließend auch beim Eindrehen der Schenkelhalsschraube. Aus diesem Grunde ist sowohl das Bohrwerkzeug als auch die Schenkelhalsschraube mit einer durchgehenden axialen Bohrung versehen.

Das Bohrwerkzeug weist am vorderen Ende seines länglichen Schaftes einen geeigneten Bohrabschnitt auf und am hinteren Ende einen Einspannabschnitt zur Verbindung mit einem rotierenden Eintreibwerkzeug.

Unter ungünstigen Bedingungen kann es vorkommen, daß der vergleichsweise dünne Kirschner-Draht beim Einbringen in den Schenkelhals verbogen wird. Es ist daher erforderlich, während des Bohrens und während des Einschraubens der Schenkelhalsschraube die Lage des Kirschner-Drahts unter Röntgenkontrolle zu überwachen. Dadurch wird verhindert, daß der Draht ungewollt in das Hüftgelenk vorgetrieben wird, weil der verbogene Abschnitt in der Bohrung des Bohrers oder der Schenkelhalsschraube klemmt. Dies muß unbedingt vermieden werden.

Aus WO 00/45714 ist ein chirurgisches Räumwerkzeug bekannt geworden, bei dem eine Antriebswelle für das Räumwerkzeug in einem äußeren flexiblen Rohr angeordnet ist. Durch zwei Anschlüsse am äußeren Rohr können Flüssigkeit eingetragen und Späne abgesaugt werden. Die Antriebswelle ist hohl für die Aufnahme eines Führungsdrahtes. Am hinteren Ende sind Anschlüsse für die Wasserzufuhr bzw. eine Absaugvorrichtung vorgesehen, und die Welle weist im Bereich des Wasserzuführanschlusses eine Öffnung auf, wodurch Wasser zwischen dem Führungsdraht und der Innenwandung der Welle strömen kann in Richtung Räumkopf, der seinerseits hohl ausgebildet ist.

Der Erfindung liegt die Aufgabe zugrunde, ein Bohrwerkzeug für einen Knochen, insbesondere den proximalen Femur, zu schaffen, mit dem der Operateur auch ohne Röntgenkontrolle erkennen kann, ob der Kirschner-Draht beim Vortreiben des Bohrwerkzeugs klemmt.

Diese Aufgabe wird durch die Merkmale des Patentanspruchs 1 gelöst.

Beim erfindungsgemäßen Bohrwerkzeug weist der Schaft im hinteren Abschnitt eine radiale Öffnung auf, die mit der axialen Bohrung verbunden ist. Vorzugsweise sind zwei diametral gegenüberliegende Öffnungen vorgesehen, die mit der axialen Bohrung in Verbindung stehen.

Nach einer weiteren Ausgestaltung der Erfindung ist die Öffnung länglich geformt und erstreckt sich parallel zur Achse des Schaftes. Vorzugsweise liegt die Öffnung nahe am Einspannabschnitt des Schaftes, zwischen einem auf dem Schaft befestigbaren, jedoch bewegbaren Schieber zur Einstellung der Bohrtiefe und dem Einspann abschnitt.

Über die Öffnung, die beim Bohrvorgang außerhalb des Knochens liegt und von außen einsehbar ist, kann der Operateur erkennen, ob während des Vortriebs des Bohrwerkzeugs eine Relativbewegung zwischen Bohrwerkzeug und Kirschner-Draht stattfindet. Ist dies nicht der Fall, weiß der Operateur, daß der Kirschner-Draht in der Bohrung des Werkzeugs klemmt und daher durch einen anderen ersetzt werden muß. Die mindestens eine Öffnung ist somit ein Sichtfenster, über das der Chirurg Anschluß bekommt über die Position des Führungsdrahtes oder stiftes.

Mit Hilfe einer länglichen Öffnung kann außerdem erkannt werden, wie weit der Kirschner-Draht im Schenkelhals eingetrieben ist, da üblicherweise die jeweilige Tiefe des Bohrwerkzeugs im Knochen ermittelt wird. Verändert sich die Position des Kirschner-Drahtes relativ zum Bohrwerkzeug in einem kritischen Ausmaß, ist der Bohrvorgang zu beenden und ein anderer Kirschner-Draht einzusetzen.

Die Erfindung wird nachfolgend anhand eines in Zeichnungen dargestellten Ausführungsbeispiels näher erläutert.
- Fig. 1: zeigt eine Seitenansicht eines Bohrwerkzeugs nach der Erfindung,
- Fig. 2: zeigt perspektivisch das hintere Ende des Bohrwerkzeugs nach Fig. 1.

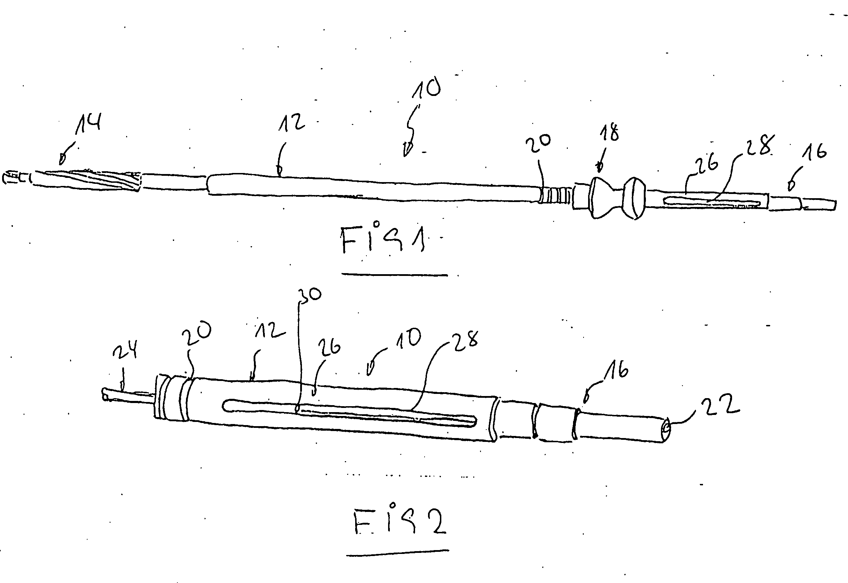

In den Fign. 1 und 2 ist ein Bohrwerkzeug 10 dargestellt mit einem länglichen Schaft 12, der am vorderen Ende einen zweistufigen Bohrabschnitt 14 aufweist, auf den nicht weiter eingegangen werden soll. Das Bohrwerkzeug 10 dient zum Aufbohren eines Schenkelhalses einer frakturierten proximalen Femur. Am hinteren Ende ist der Schaft 12 mit einem Einspannabschnitt 16 versehen. Ein rastend auf dem Schaft 12 festlegbarer jedoch bewegbarer Schieber 18 dient zur Einstellung der gewünschten Bohrtiefe. Es sind einzelne radiale Nuten 20 zu erkennen, in denen der Schieber 18 verrasten kann und die die Bohrtiefe anzeigen. In Fig. 2 ist der Schieber 18 fortgelassen.

Man erkennt, daß das Werkzeug 10 eine durchgehende axiale Bohrung 22 aufweist. Sie dient zur Aufnahme eines Kirschner-Drahtes 24, der abschnittsweise in Fig. 2 dargestellt ist. In einem Abschnitt 26 des Schaftes 12 nahe dem, Einspannabschnitt 16 sind diametral gegenüberliegend zwei achsparallele längliche Schlitze geformt, von denen einer bei 28 zu erkennen ist. Die Schlitze 28 stehen mit der axialen Bohrung 22 in Verbindung. Daher ist das Ende des Kirschner-Drahtes 24, das bei 30 innerhalb der axialen Bohrung 22 sich befindet, gut sichtbar. Beim Bohrvorgang kann daher der Operateur die Relativlage der Kirschner-Drahtes 24 bzw. des freien Endes 30 gut beobachten und feststellen, ob der Schaft 12 sich relativ zum Kirschner-Draht 24 bewegt oder dieser mit vorgetrieben wird.

Statt einer Öffnung können selbstverständlich auch mehrere vorgesehen werden, die hintereinander oder auch in Umfangsrichtung versetzt angeordnet sein können.

## Patentansprüche

1. Bohrwerkzeug für einen Knochen, insbesondere den proximalen Femur, das einen länglichen Schaft (12), einen Bohrabschnitt (14) am vorderen Ende und einen Einspannabschnitt (16) am hinteren Ende des Schaftes (12) aufweist, wobei das Werkzeug (10) eine durchgehende axiale Bohrung (22) aufweist für die Aufnahme eines Führungsdrahtes (24), und der Schaft (12) in einem hinteren Abschnitt (26) eine radiale Öffnung (28) aufweist, die mit der axialen Bohrung (22) verbunden ist, **dadurch gekennzeichnet, dass** die Öffnung (28) als während des Bohrvorgangs von außen einsehbares Sichtfenster angeordnet ist.

2. Bohrwerkzeug nach Anspruch 1, **dadurch gekennzeichnet, dass** auf diametral gegenüberliegenden Seiten des Schaftes (12) mit der axialen Bohrung (22) verbundene Öffnungen (28) geformt sind.

3. Bohrwerkzeug nach Anspruch 1 oder 2, **dadurch gekennzeichnet, dass** eine längliche achsparallele Öffnung (28) vorgesehen ist.

4. Bohrwerkzeug nach einem der Ansprüche 1 bis 3, **dadurch gekennzeichnet, dass** die Öffnung (28) zwischen einem auf dem Schaft (12) befestigbaren, jedoch bewegbaren Schieber (18) zur Einstellung der Bohrtiefe und dem Einspannabschnitt (16) geformt ist.

## Claims

1. A boring tool for a bone, particularly the proximal femur, which has an elongate shank (12), a boring portion (14) at the front end, and a locking portion (16) at the rear end of the shank (12) wherein the tool (10) has an axial through bore (22) for receiving a guide wire (24) and the shank (12), in a rear portion (26), has one radial aperture (28) which is connected to the axial bore (22), **characterized in that** the aperture (28) is disposed as a window which can be looked over from outside during the boring procedure.

2. The boring tool as claimed in claim 1, **characterized in that** diametrically opposed sides of the shank (12) have formed thereon apertures (28) which are connected to the axial bore (22).

3. The boring tool as claimed in claim 1 or 2, **characterized in that** one elongate, axially parallel aperture (28) is provided.

4. The boring tool as claimed in any one of the claims 1 to 3, **characterized in that** the aperture (28) is formed between a movable slide (18) which can be fixed to the shaft (12) for setting the boring depth, and the locking portion (16).

## Revendications

1. Outil de perçage pour un os, en particulier pour le fémur proximal, qui présente une tige longitudinale (12), un segment de perçage (14) à l'extrémité antérieure et un segment de serrage (16) à l'extrémité postérieure de la tige (12), l'outil (10) présentant un perçage (22) axial traversant pour recevoir un fil de guidage (24) et la tige (12) présentant une ouverture (28) radiale dans un segment postérieur (26), ouverture qui est raccordée au perçage (22) axial, **caractérisé en ce que** l'ouverture (28) est agencée comme une fenêtre de contrôle visible de l'extérieur pendant le processus de perçage.

2. Outil de perçage selon la revendication 1, **caractérisé en ce que** des ouvertures (28) raccordées au perçage (22) axial sont formées sur les côtés diamétralement opposés de la tige (12).

3. Outil de perçage selon la revendication 1 ou 2, **caractérisé en ce qu'**une ouverture (28) longitudinale parallèle à l'axe est prévue.

4. Outil de perçage selon l'une des revendications 1 à 3, **caractérisé en ce que** l'ouverture (28) est formée entre un coulisseau (18) pour le réglage de la profondeur de perçage, pouvant être fixé sur la tige (12) et pouvant toutefois se déplacer sur celle-ci, et le segment de serrage (16).
